(19) Europäisches Patentamt
European Patent Office
Office européen des brevets

(11) Veröffentlichungsnummer: **0 080 079 B1**

(12) # EUROPÄISCHE PATENTSCHRIFT

(45) Veröffentlichungstag der Patentschrift: **25.06.86**

(51) Int. Cl.[4]: **C 07 D 233/78**, C 07 D 233/76, C 08 F 22/38

(21) Anmeldenummer: **82110068.2**

(22) Anmeldetag: **02.11.82**

(54) **Verfahren zur Herstellung von Hydantoinen.**

(30) Priorität: **11.11.81 DE 3144701**
**11.11.81 DE 3144697**

(43) Veröffentlichungstag der Anmeldung: **01.06.83 Patentblatt 83/22**

(45) Bekanntmachung des Hinweises auf die Patenterteilung: **25.06.86 Patentblatt 86/26**

(84) Benannte Vertragsstaaten: **AT DE FR GB IT**

(56) Entgegenhaltungen:
**EP - A - 0 002 662**
**EP - A - 0 033 477**
**DE - A - 2 718 102**

(73) Patentinhaber: **BAYER AG, Konzernverwaltung RP Patentabteilung, D-5090 Leverkusen 1 Bayerwerk (DE)**

(72) Erfinder: **Schulte, Bernhard, Dr., Südwall 80 A, D-4150 Krefeld (DE)**
Erfinder: **Jakob, Wolfgang, Am Domacker 81, D-4130 Moers 1 (DE)**
Erfinder: **Dünwald, Willi, Dr., Geschwister-Scholl-Strasse 16, D-5090 Leverkusen 1 (DE)**
Erfinder: **Meyer, Karl-Heinrich, Dr., Deswatinesstrasse 89, D-4150 Krefeld (DE)**
Erfinder: **Zecher, Wilfried, Treptower Strasse 6, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von Verbindungen mit mindestens einem Hydantoinring im Molekül aus Carbodiimiden und bestimmten Derivaten α,β-ungesättigter Carbonsäuren.

Hydantoine, Polyhydantoine und Verfahren zu ihrer Herstellung sind bekannt (vergl. Am. Chem. J. 45, 383; BE-PS 678 282; DE-OS 2 714 655).

Aus EP-A-2662 und EP-A-33 477 ist die Herstellung von Hydantoinen durch Umsetzung von Isocyanaten mit α,β-ungesättigten Carbonsäuren oder deren Derivaten bekannt.

Niedermolekulare Hydantoine werden bevorzugt im Pharma- und Pflanzenschutzbereich verwendet, höhermolekulare Hydantoine sind beispielsweise im wärmebeständigen Beschichtungsmittelsektor von Bedeutung (FR-PS 1 484 694).

Gegenstand der Erfindung ist ein neues Verfahren zur Herstellung von Hydantoinringe enthaltenden Verbindungen, das dadurch gekennzeichnet ist, dass man ein Carbodiimid mit einem Derivat einer α,β-ungesättigten Carbonsäure der Formel

$$HOOC{-}CH=CH{-}R^5$$

worin

$R^5$ Benzoyl ($COC_6H_5$), $COOR^6$ oder eine Säureamidgruppe $-CONR^7R^8$ bedeutet,

worin

$R^6$ Benzyl, Alkyl, Cycloalkyl, Alkenyl und Alkinyl bedeutet und

$R^7$ und $R^8$ Alkyl bedeuten,

bei Temperaturen von $-20$ bis 250 °C, gegebenenfalls in Anwesenheit eines Katalysators und gegebenenfalls in Anwesenheit eines Lösungsmittels umsetzt.

Es ist bekannt [Chem. Rev. 67,2 (1967) S. 107], dass die Umsetzung von Carbodiimiden mit Carbonsäuren zu mehreren Reaktionsprodukten führt, vorwiegend zu Acylharnstoffen, Carbonsäureanhydriden und Harnstoffen. So ist auch die Umsetzung von Fumarsäure mit Carbodiimid zum entsprechenden über die Doppelbindung verknüpften Diacylharnstoff und die Reaktion von Maleinsäure mit Carbodiimid zum Maleinsäureanhydrid [J. Prakt. Chem. 79, 513 (1909)] beschrieben. Von F. Zetzsche und G. Röttger wurde sogar ein Nachweis für α,β-ungesättigte Carbonsäuren als Ureide mit Hilfe der Carbodiimide angegeben [Chem. Ber. 72, 1599 (1939]], wobei die Bildung der N-Acyl-N,N′-bis(4-dimethylaminophenyl)-harnstoffe aus Di-(4-dimethylamino-phenyl)-carbodiimid und einigen α,β-ungesättigten Carbonsäuren festgestellt wurde. Hydantoine werden so nicht gebildet.

Überraschenderweise gelingt die Hydantoinbildung, wenn man nach den erfindungsgemässen Bedingungen arbeitet und statt der Carbonsäuren ausgewählte Derivate ungesättigter Carbonsäuren einsetzt.

Als Carbodiimid-Verbindungen im Sinne der Erfindung können Monocarbodiimide mit einer $-N=C=N$-Gruppe im Molekül, deren cyclische Dimere oder Trimere oder auch lineare bzw. verzweigte Polycarbodiimide mit mehr als zwei Carbodiimid-Gruppen im Molekül verwendet werden.

Bevorzugt sind Carbodiimide der Formeln I und II

$$R^1{-}N=C=N{-}R^2 \qquad \text{(I)} \qquad \qquad [Y{-}N=C=N]_n \qquad \text{(II)}$$

in denen

$R^1$ und $R^2$ gleich oder verschieden einen aliphatischen Rest mit 1–20 C-Atomen, einen cycloaliphatischen Rest mit 5–12 C-Atomen, einen aliphatisch-aromatischen Rest mit 6–20 C-Atomen, einen aromatischen Rest mit 6–16 C-Atomen, einen Heteroatome wie N, O oder S enthaltenden aromatischen oder cycloaliphatischen Rest mit 5–12 C-Atomen, die jeweils gegebenenfalls mit Halogen (Chlor, Brom, Iod, Fluor), Nitril-,
$C_2$–$C_{12}$-Dialkylamino, $C_7$–$C_{12}$-Alkylarylamino,
$C_2$–$C_{18}$-Alkoxycarbonyl, $C_7$–$C_{18}$-Aroxycarbonyl,
$C_2$–$C_{18}$-Alkylcarboxy, $C_7$–$C_{18}$-Arylcarboxy,
$C_1$–$C_{18}$-Alkoxy, $C_6$–$C_{18}$-Aroxy bzw. Diarylamino,
$C_1$–$C_{18}$-Alkyl-, Halogenalkyl-
mit $C_1$–$C_{18}$, Nitrogruppen
substituiert sein können oder einen
$C_2$–$C_{12}$-Dialkylamino, $C_1$–$C_{18}$-Alkylamino-,
$C_2$–$C_{18}$-Alkoxycarbonyl-, $C_6$–$C_{18}$-Glykosylrest
oder eine $-Si(R^6)_3$-, $-Sn(R^6)$-, $-SO_2R^6$-Gruppe (mit $R^6$ = $C_6$–$C_{12}$-Aryl bzw. $C_1$–$C_8$-Alkyl) bedeuten oder miteinander als Glieder entsprechend cyclischer organischer Reste verknüpft sein können und

Y die für $R^1$, $R^2$ angegebene Bedeutung hat und dabei bevorzugt für aliphatische Reste mit 2–12 C-Atomen, cycloaliphatische Reste mit 5–12 C-Atomen oder Arylreste mit $C_6$–$C_{16}$- oder über O, S, $SO_2$, $CH_2$, $CH_3{-}C{-}CH_3$ oder CO verknüpfte Diphenylreste oder für $-Si(R^6)_2$-, $Su(R^6)_2$-Gruppen steht, und

n eine ganze Zahl von 2–2000, vorzugsweise 2 bis 1000 bedeutet.

Als Monocarbodiimide werden erfindungsgemäss N, N′-symmetrisch und/oder asymmetrisch substituierte aliphatische, aliphatisch-aromatische, cyclische, heterocyclische, aromatische, gegebenenfalls durch Heteroatome substituierte Verbindungen mit einer $-N=C=N$-Gruppe im Molekül eingesetzt, z.B. Dialkylcarbodiimide wie
Dimethyl-, Diethyl-, Diisopropyl-, Dihexyl-,
Dibutyl-, Dinonyl-, Didodecyl- und
Distearylcarbodiimid,
vorzugsweise aromatische, gegebenenfalls substituierte Monocarbodiimide wie
Diphenyl-, Ditolyl-, Dinaphthylcarbodiimid,
Di-(p-iodphenyl)-, Di-(p-dimethylaminophenyl)-,
Di-(-pyridyl)-, Di-Nitro-, -Alkoxy-, -Aryloxy-,
-Chlor-, -Dichlor-, -Trichlor-, -Tetrachlor-,
-Pentachlor-, Benzyl-,
-p-Bromphenylcarbodiimid oder
Carbodiimid-dibenzoesäureester,
-di-phthalsäureester,
-di-isophthalsäureester,
Carbodiimid-dibenzonitril,
cycloaliphatische Carbodiimide wie

Dicyclohexylcarbodiimid und ungesättigte Carbodiimide wie Diallyl-, Dioleyl-, Dicyclohexenylcarbodiimid.

Diese Carbodiimid-Verbindungen können nach bekannten Verfahren z.B. aus den entsprechenden Thioharnstoffen in Gegenwart von Metalloxiden, Quecksilbersalzen, Natriumsalzen, Arylsulfochloriden bzw. durch Oxidation von Thioharnstoffen oder aus S-Alkylisothioharnstoffen, Harnstoff-Verbindungen, wie z.B. in Chem. Rev. 67,2 (1967), S. 107 angegeben oder aus den entsprechenden Isocyanat-Verbindungen unter Kohlendioxidabspaltung in Gegenwart der bekannten speziellen Katalysatoren zur $CO_2$-Abspaltung hergestellt werden (FR-PS 1 180 307).

Weiterhin können die N-Sulfonylcarbodiimide $RSO_2N=C=NR$, die N-Aminocarbodiimide $RN=C=NR_2$ oder die N, N'-Disilylcarbodiimide, wie diese z.B. in Chem. Rev. 67,2 (1967), S. 107 aufgeführt sind, eingesetzt werden.

Als erfindungsgemäss einzusetzende Ausgangskomponenten kommen ebenso aliphatische, cycloaliphatische, araliphatische, aromatische und heterocyclische lineare oder verzweigte Polycarbodiimide mit mehr als 2 Carbodiimidgruppen in Betracht, sowie deren Gemische oder Polycarbodiimide, die eine statistische Zusammensetzung oder einen blockartigen Aufbau aus unterschiedlichen Strukturelementen in bestimmter Sequenzlänge im Polymermolekül aufweisen und somit die oben angeführten aliphatischen, cycloaliphatischen, araliphatischen, aromatischen und heterocyclischen Struktureinheiten in unterschiedlichsten Verhältnissen sowohl statistisch verteilt als auch blockweise angeordnet im Polymermolekül enthalten können.

Im Falle der Synthese dieser Polycarbodiimide mit zwei oder mehr Carbodiimidgruppen im Molekül aus mehrfunktionellen Isocyanaten können die aus der Literatur bekannten Katalysatoren (vgl. z.B. FR-PS 1 180 307), beispielsweise Phospholine, Phospholidinsulfide usw. oder auch metallorganische Verbindungen der Gruppe Ia–IIIa, wie z.B. Phenyllithium, Diethylzink eingesetzt werden.

Dabei können die erfindungsgemässen Polycarbodiimid-Verbindungen aus Polyisocyanaten, wie sie z.B. in Annalen 562, S. 75–136; Am.Chem. J. 45, 383; DE-OS 2 714 655; US-PS 3 397 253; EP-PS 0 012 379 umfassend aufgeführt sind, hergestellt werden.

Besonders bevorzugt sind Gemische aus Poly-Toluylencarbodiimiden (2,4- und 2,6-Substitutionsprodukte), Poly-m-phenylencarbodiimiden, sowie Polycarbodiimide auf der Basis von Anilin-Formaldehydkondensaten mit Polyphenylenmethylen-Struktur und die Poly-4,4'-diphenylether-, Poly-p-phenylen-, Poly-1,5-naphthylencarbodiimide, Polyisophoron-carbodiimide und Polyhexamethylencarbodiimide und/oder deren Gemische, sowie Blockpolycarbodiimide z.B. der folgenden Strukturen:

–B–B–B–A–A–A–A–B–B–B–
–C–C–B–B–B–A–A–A–A–B–B–B–C–C–

wobei A z.B. ein aromatisches Strukturelement wie Diphenylmethan ist, B einen aliphatischen Rest R wie z.B. den Isophoronrest und C eine aromatische Einheit wie z.B. die Toluylen- oder Naphthylengruppe darstellt. Diese Blockpolycarbodiimide können z.B. hergestellt werden, indem die Carbodiimidisierung der einzelnen verwendeten mehrfunktionellen Isocyanate stufenweise nacheinander erfolgt. Die aufgeführten Strukturen und technisch leicht zugänglichen bisfunktionellen Isocyanate verdeutlichen die Variationsbreite im Hinblick auf Sequenzlängen und Mengenverhältnisse der einzelnen Bausteine, wobei die Polycarbodiimide auch gezielt verzweigt werden können, wenn z.B. tri- und mehrfunktionelle Isocyanate in den Carbodiimidisierungsstufen eingestuft werden.

Geeignete Derivate α,β-ungesättigter Carbonsäuren entsprechen der allgemeinen Formel III

$$HOOC{-}CH{-}CH{-}R^5 \qquad (III),$$

worin

$R^5$ Benzoyl ($COC_6H_5$), $COOR^6$ oder eine Säureamidgruppe $-CONR^7R^8$ bedeutet,

worin

$R^6$, $R^7$ und $R^8$ Alkyl (bevorzugt $C_1$–$C_{20}$ wie Methyl, Ethyl, iso-Propyl, Hexyl, Undecyl, Eicosyl) und

$R^6$ ausserdem Benzyl Cycloalkyl (bevorzugt $C_5$–$C_{10}$ wie Cyclopentyl, Cyclohexyl, Cycloheptyl, Cyclodecyl), Alkenyl (bevorzugt $C_2$–$C_{20}$ wie Allyl, Butenyl, Pentenyl, Decenyl, Eicosenyl), Alkinyl (bevorzugt $C_2$–$C_{20}$ wie Propargyl, Butinyl, Pentinyl, Hexinyl, Octinyl, Eicosinyl), bedeuten.

Beispiele für geeignete α,β-ungesättigte Carbonsäure-Derivate sind:

die Halb-Amide der Fumar- und Maleinsäure, besonders bevorzugt die N-Dimethyl-, N-Diethyl-, N-Methylethylmaleinamidsäure bzw. -fumaramidsäure, die Monoester der Malein- und Fumarsäure, wie z.B. Fumar- bzw. Maleinsäuremonomethylester, -ethylester, die z.B. durch Umsetzung der Malein-, Fumarsäure oder des Maleinsäureanhydrids mit den entsprechenden Alkoholen wie Butanol, Isopropanol, Hexanol, Cyclohexanol, Benzylalkohol, Allylalkohol, Propargylalkohol oder Undecylalkohol usw. zu den Monoestern umgesetzt werden, sowie β-Benzoylacrylsäure, die z.B. durch Friedel-Crafts-Acylierung gewonnen werden kann.

Die erfindungsgemässe Umsetzung lässt sich beispielhaft durch folgende Gleichung wiedergeben:

$$R^1{-}N=C=N{-}R^2 \quad + \quad HOOC{-}CH=CH{-}R^5 \quad \rightarrow$$

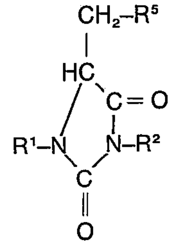

oder $-\!\!\left[y{-}N=C=N\right]_n\!\!-$ +

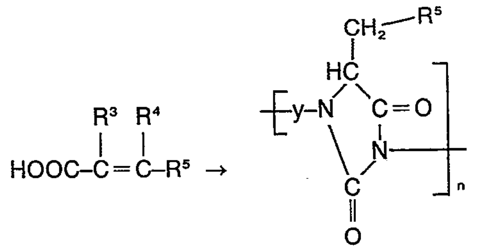

Im allgemeinen werden dementsprechend pro Äquivalent Carbodiimid mindestens 1 Val. des α,β-ungesättigten Carbonsäurederivates verwendet, doch sind auch sehr weitgehende Abweichungen von diesen Mengenverhältnissen möglich.

Die erfindungsgemässen in 5-Stellung substituierten Hydantoine können eindeutig durch IR-Spektren anhand der charakteristischen Banden für Hydantoine und Ester, bzw. mit Hilfe der NMR-Spektroskopie identifiziert werden. Die höhermolekularen Hydantoine weisen Lösungsviskositäten von 50–100.000 mPas, vorzugsweise 100–50.000 mPas auf, die an einer 15gewichtsprozentigen Lösung in m-Kresol 70 bis 20 °C bestimmt werden.

Die erfindungsgemässe Umsetzung kann in homogener Phase, wobei die Lösungsmittel unter den Reaktionsbedingungen nicht reagieren oder gegebenenfalls lockere, weiterreagierende Additionsverbindungen bilden oder als heterogene Reaktion in Suspension oder in Substanz oder in einem Überschuss einer der Reaktionskomponenten durchgeführt werden.

Als Reaktionsmedien eignen sich inerte organische Flüssigkeiten, z.B. aliphatische, aromatische oder heterocyclische, gegebenenfalls substituierte, Kohlenwasserstoffe wie Chlorkohlenwasserstoffe, z.B. Tetrachlorkohlenstoff, Methylenchlorid, Chloroform, Ethylenchlorid, Tetrachlorethan oder Cyclohexan, Dioxan, Tetrahydrofuran, Ligroin oder Chlorbenzol, o-Dichlorbenzol, Xylol, Toluol, Carbitol, Dimethyl-, Diethylether, wobei besonders für die Herstellung von Verbindungen mit nur einem Hydantoinring im Molekül Kombinationen aus einer niedrig- und einer höhersiedenden Flüssigkeit, z.B. Gemische aus Methylenchlorid/Chlorbenzol bzw. o-Dichlorbenzol oder Methylenchlorid/Toluol bzw. Xylol bevorzugt werden. Dabei kann die leichterflüchtige Komponente im Verlauf der Reaktion durch Abdestillieren zur Wärmeabfuhr genutzt werden, und in der höhersiedenden Komponente wird die Reaktion zum Hydantoin zum Abschluss gebracht. Für die Herstellung der erfindungsgemässen Polyhydantoine sind Kohlenwasserstoffe, Halogenkohlenwasserstoffe, aromatische Hydroxyverbindungen wie Phenole und Kresole, Ester, Ketone, Sulfoxide und Solfone, Ether substituierte Amide, Nitrile geeignet. Beispiele dafür sind

Xylole, o-Dichlorbenzol, Acetophenon,
Cyclohexanon, Propylencarbonat,
ε-Caprolactam, Dimethylsulfoxid,
Glykolmonomethyletheracetat, γ-Butyrolacton,
ε-Caprolacton, Benzoesäurealkylester,
N-Methylpyrrolidon, Dimethylformamid,
Dimethylacetamid, Benzonitril

sowie deren Gemische oder Gemische mit Benzylalkohol.

Bevorzugt werden binäre oder besonders ternäre Gemische aus den vorgenannten Lösungs- bzw. Verdünnungsmitteln mit den letztgenannten Verbindungen eingesetzt, wie z.B. Methylenchlorid/Toluol bzw. Xylol/Phenol bzw. Kresol oder γ-Butyrolacton, wobei die am leichtesten flüchtige Komponente zur Wärmeabfuhr oder als gutes Lösungsmittel für die Polycarbodiimide geeignet ist, die höhersiedende Verbindung nach Abschluss der Reaktion zum Polyhydantoin noch teilweise oder auch vollständig in der Lacklösung verbleiben kann, wie z.B. Toluol, Xylol, Solvesso, und diese Komponenten somit bevorzugt in der Elektroisoliertechnik gebräuchliche Verschnittmittel darstellen, und die am schwersten flüchtige Komponente wie Kresol, Phenol die eigentlichen Lösungsmittel für das Polyhydantoin-Reaktionsprodukt darstellen.

Als Verschnittmittel sind ausserdem aliphatische und aromatische Kohlenwasserstoffe wie Cyclohexan, Xylol, Toluol und deren technische Gemische geeignet.

Die erfindungsgemässe Umsetzung kann in Gegenwart von Katalysatoren, die die Addition von OH-funktionellen Verbindungen an die Carbodiimidgruppe beschleunigen, wie z.B. Kupfer-I-, Kupfer-II-chlorid usw. oder auch unter Zusatz der für die Malein-Fumarsäureumlagerung geeigneten Katalysatoren, wie z.B. lod oder unter Verwendung der für die Cyclisierung zum Hydantoinring bekannterweise geeigneten Katalysatoren wie Basen, beispielsweise Triethylamin, N-Methylmorpholin, Endoethylenpiperazin, wie Säuren, beispielsweise p-Toluolsulfonsäuren, wie Alkohole wie Methanol, Phenol, wie Metalle, insbesondere von Eisen, Blei, Zink, Zinn, Kupfer, Kobalt, Titan, Mangan, beispieslweise Titantetrabutylat, Titanaminoalkohol, Eisenacetylacetonat, Dibutylzinnlaurat, Bleiacetat, Zinkoctoat oder in Gegenwart starker Basen wie Kalium-tert.-butylat, Natriummethylat durchgeführt werden.

Insbesondere geeignet sind die Katalysatoren, die zur Bildung der Carbodiimide benutzt werden, z.B. Phospholinoxid.

Im Falle der Verwendung von Polycarbodiimiden, die aus mehrfunktionellen Isocyanaten in bekannter Weise hergestellt worden sind, kann direkt das Reaktionsgemisch einschliesslich des darin enthaltenen Katalysators benutzt werden.

Zur Durchführung der erfindungsgemässen Verfahren werden die Carbodiimide, vorzugsweise gelöst oder suspendiert in den oben angegebenen Lösungs-/Verdünnungsmittelkombinationen, mit den α,β-ungesättigten Carbonsäuren in den angegebenen Reaktionsmedien einige Minuten bis zu mehreren Stunden bei Temperaturen von −20 °C bis 250 °C, bevorzugt 30 bis 200 °C, gehalten.

Der Reaktionsverlauf kann über die IR-Spektren verfolgt werden. In der Anfangsphase ist die Reaktion sehr exotherm, so dass die Reaktionskomponenten vorzugsweise bei niedrigen Temperaturen

von 20 °C bis 50 °C vereinigt werden oder eine Reaktionskomponente portionsweise eingetragen wird, vorzugsweise bei 20 bis 45 °C. Dabei können sowohl die Carbodiimide als Lösungen oder Suspensionen als auch die α,β-ungesättigten Carbonsäure-Derivate, gegebenenfalls in den angegebenen Reaktionsmedien, vorgelegt werden.

Vorteilhafterweise werden im Hinblick auf eine technische Polyhydantoinherstellung im Falle der Carbodiimidisierung von mehrfunktionellen Isocyanaten die Carbodiimide vorgelegt, da sie nach Abschluss ihrer Herstellung sofort weiter zum erfindungsgemässen Hydantoin umgesetzt werden können.

Werden Polycarbodiimide eingesetzt, die einen blockartigen Aufbau –B–A–B– aufweisen, wobei A und B Sequenzen von jeweils verschiedenen Kettengliedern darstellen, wird im allgemeinen so verfahren, dass ein Diisocyanat (z.B. 4,4'-Diisocyanatdiphenylmethan) in den aufgeführten, für die Herstellung von Monohydantoinen geeigneten Lösungs- bzw. Verdünnungsmittelkombinationen aus einer leicht- und schwerer flüchtigen Flüssigkeit, wie z.B. Methylenchlorid/Toluol, bis zum nahezu vollständigen Umsatz carbodiimidisiert wird. Anschliessend wird gegebenenfalls unter Zusatz von weiterem Lösungs-/Verdünnungsmittel das Diisocyanat – oder auch z.B. Triisocyanat – mit der Struktureinheit B (z.B. Isophoron-, Tolyl-) zugegeben und die Carbodiimidisierung fortgesetzt, wobei dann mit Vorteil Monoisocyanate (z.B. Phenyl-, Tolyl-, Naphthyl-, Cyclohexyl-, Methyl-, Cyclohexenyl-, Oleylisocyanat-, Isocyanatobenzoesäureester, -phthalsäureester, oder -isophthalsäureester) im Verlauf des weiteren Polycarbodiimidaufbaues als Regler eingetragen werden.

Anschliessend können die α,β-ungesättigten Carbonsäure-Derivate in Substanz oder gelöst in den geeigneten geschilderten Reaktionsmedien unter Berücksichtigung des sehr exothermen Verlaufs zur Reaktion gebracht werden. Dabei wird der Reaktionsverlauf zum Hydantoin mit Vorteil durch stufenweise Steigerung der Reaktionstemperatur erreicht. Es können Inhibitoren, wie z.B. Tolylhydrochinon zugesetzt werden, um eine eventuell einsetzende Polymerisation der Doppelbindung zu vermeiden.

Bevorzugt wird die erfindungsgemässe Umsetzung der Polycarbodiimide mit α,β-ungesättigten Carbonsäure-Derivaten in Gegenwart von phenolischen Verbindungen wie z.B. Phenol, Kresol und deren Gemischen oder γ-Butyrolacton, N-Methylpyrrolidon oder in Gemischen aus den letztgenannten Lösungsmitteln mit Benzylalkohol durchgeführt, mit dem Vorteil, dass diese Reaktionsmedien auch Lösungsmittel für die polymeren Hydantoine sind.

Die Umsetzung wird zweckmässigerweise unter einem inerten Schutzgas wie $N_2$ oder Argon durchgeführt.

Die erfindungsgemässe Reaktion kann kontinuierlich oder diskontinuierlich bei Normaldruck oder bei Überdruck ausgeführt werden.

Die Aufarbeitung der niedermolekularen Reaktionsprodukte kann nach gängigen Methoden wie z.B. Kristallisation erfolgen.

Die nach dem erfindungsgemässen Verfahren zugänglichen monomolekularen Hydantoine zeigen Wirkungen auf dem pharmazeutischen und Pflanzenschutzsektor.

Die erfindungsgemässen Polyhydantoine zeichnen sich durch besonders Temperaturbeständigkeit sowie eine gute Löslichkeit und ausgezeichnete Verlaufseigenschaften bei der Drahtbeschichtung aus und sind geeignet zur Herstellung von Klebern, Lacken, Folien und Formkörpern.

Vorzugsweise sind die Hydantoingruppen enthaltenden Produkte für Einbrennlacke, insbesondere Draht- bzw. Elektroisolierlacke geeignet, wobei der Festgehalt der möglichen Lacklösungen in weiten Grenzen schwanken kann und vorzugsweise im Bereich von 20–80 Gew.-% liegt, und zwar abhängig vom beabsichtigten Einsatzzweck.

Weiterhin können mit Hilfe des erfindungsgemässen Verfahrens bekannte temperaturbeständige Kunststoffe bzw. Lackrohrstoffe, wie z.B. Polyamide, Polyester, Polyurethane, Polyesteramide, Polyhydantoine, Polyamidimide, Polyesterimide usw. durch Hydantoingruppen der beschriebenen Art modifiziert werden.

Beispiel 1

In 70 g Toluol und 70 g Chlorbenzol werden 75 g 4,4'-Di-isocyanatodiphenylmethan und 2 g Phenylisocyanat gelöst. Nach Zugabe von 0,5 g Phospholinoxid (Gemisch aus
1-Methyl-1-phospha-2-cyclopenten-1-oxid- und
1-Methyl-1-phospha-3-cyclopenten-1-oxid)
wird unter Gasuhr-Anschluss innerhalb von 2½ Std. auf 60–65 °C aufgeheizt.

Der Polycarbodiimidsuspension werden bei 45 °C 100 g Phenol zugesetzt, und bei 25 °C wird eine Lösung von 44 g Maleinsäuremonomethylester (Gesamtsäurezahl 422, Teilsäurezahl 396) in 60 g Phenol eingerührt, wobei die Temperatur auf 75–80 °C ansteigt. Dann wird innerhalb von 4 Std. auf 180 °C unter Abdestillieren von Toluol/Chlorbenzol erhitzt. Nach ½ Std. 180 °C werden 40 g m-Kresol 70 und nach weiteren 2 Std 35 g m-Kresol 70 zugegeben. Nach einer weiteren ½ Std. bei 180 °C wird auf 140 °C abgekühlt, es wird mit 15 g Xylol verdünnt, und man erhält eine klare braunrote Polymerlösung mit einem Festgehalt von 31,3% (5 Minuten bei 350 °C eingebrannt) und einer Viskosität von 1520 mPas (15%ig mit m-Kresol 70 verdünnt bei 20 °C in einem Höppler-Viskosimeter gemessen). Das IR-Spektrum dieser Polyhydantoinesterlösung zeigt die entsprechenden Banden bei 1770, 1710 und 1415 $cm^{-1}$. Nach dem Ausfällen des Polymeren aus $CH_3OH$ werden die für Hydantoinstrukturen typischen Banden im IR bei 1770–1775, 1720 und 1410 $cm^{-1}$ festgestellt. (Chlorbenzol kann durch Xylol zur Herstellung des Polycarbodiimids ersetzt werden). Die Applizierung auf einen Kupferdraht als 22%ige Lösung (mit Kresol/Xylol 1:1 verdünnt: Auslaufzeit 470 sec aus einem DIN 4-Becher bei Raumtemperatur) führt zu folgenden Prüfdaten (Düsenauftragsverfahren; Cu-Draht 0,7 mm Durchmesser), wobei die für Polyhydantoine bezeichnenden guten Verlaufeigenschaften bei hohen Erweichungstemperaturen festgestellt werden.

Der in einem 4-m-Ofen bei 8–12 m pro Min. bei 400 °C Einbrenntemperatur beschichtete Cu-Lackdraht wird nach DIN 46 453 geprüft. Er besitzt eine Erweichungstemperatur von mindestens 350 °C, einen Hitzeschock von mindestens 260, gute Elastizitäten mit Aussenfaserdehnungen bis 88%, eine Filmhärte von mindestens 4 H – auch nach einer 30-minütigen Alkoholbehandlung bei 60 °C – Dauerwärmebeständigkeiten bis zu 7 Tagen bei 180 °C, eine Durchschlagfestigkeit von mindestens 7 KV und gute Chemikalienbeständigkeit.

Beispiel 2

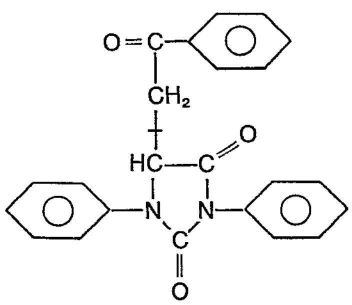

Zu 72 g einer 27%igen Diphenylcarbodiimidlösung (0,1 Mol) in Toluol wird bei Raumtemperatur eine Lösung von 17,6 g (0,1 Mol) β-Benzoylacrylsäure in 210 g Toluol innerhalb einer Stunde gegeben, danach 3 Std. gerührt. Die ausgefallenen Kristalle werden abgesaugt, mit Toluol gewaschen und getrocknet (Ausbeute 19 g).

5 g des Kristallisats werden in 30 g o-Dichlorbenzol in Anwesenheit geringer Mengen Toluhydrochinon 3 Std. am Rückfluss gekocht. Das Reaktionsgemisch wird im Vakuum eingeengt, der Rückstand aus Isopropanol umkristallisiert. Das weisse Kristallisat (4 g) zeigt im IR die Hydantoinbanden bei 1770/1710 und 1410 $cm^{-1}$ und die Carbonylbande bei 1670–1675 $cm^{-1}$. Die Struktur des 1,3-Diphenyl-5-(benzoyl-methylen)-hydantoins wird durch das NMR-Spektrum und die Elementaranalyse bewiesen.

Analyse: $C_{23}H_{18}N_2O_3$ (370)
ber.: C 74,6 H 4,9 N 7,6
gef.: C 75,0 H 4,8 N 7,6

Beispiel 3

In einem 500-ml-Dreihalskolben (Rührer, Innenthermometer, Destillationsaufsatz mit Vorlage und Gasuhranschluss) werden 50 g 4,4'-Diisocyanatodiphenylmethan und 2,5 g Phenylisocyanat in 40 g Toluol und 40 g Chlorbenzol gelöst. Nach Zugabe von 0,5 g Phospholinoxid (Gemisch aus
1-Methyl-1-phosphat-2-cyclopenten-1-oxid- und
1-Methyl-1-phospha-3-cyclopenten-1-oxid)
wird innerhalb von 1 Std. auf 60 °C aufgeheizt. Die erhaltene Polycarbodiimidsuspension zeigt die typischen Banden der Carbodiimidgruppen im IR-Spektrum bei 2140/2110 $cm^{-1}$, die Isocyanatbande wird im IR-Spektrum nicht mehr festgestellt. Durch Zugabe von 60 g m-Kresol 70 wird auf 40 °C abgekühlt. Anschliessend wurde eine Lösung von 27,5 g Maleinsäuremonomethylester in 100 g Kresol homogen eingerührt, wobei die Temperatur auf ca. 80 °C ansteigt. Nach Abfall der Temperatur auf 70 °C ist das Carbodiimid abreagiert, die Bande bei 2110–2140 $cm^{-1}$ wird im IR nicht mehr beobachtet. Innerhalb von 2 Std. wird auf 180 °C unter Destillation (Toluol/Chlorbenzol) aufgeheizt, und nach weiteren 6 Std. bei 180–185 °C erhöht man eine klare braunrote Polymerlösung mit einem Festgehalt von 33,1% (2 Std bei 200 °C) und einer Viskosität von 750 mPas (bei 20 °C 15%ig in Kresol mit einem Höppler-Viskosimeter gemessen) mit den für Hydantoine typischen IR-Banden bei 1765, 1710 und 1430 $cm^{-1}$ in m-Kresol 70. Die Applikation auf Kupferdraht von 0,7 mm Durchmesser mit dieser auf einen Festgehalt von 25% mit Xylol verdünnten Lacklösung zeigt die für Polyhydantoine charakteristischen ausgezeichneten Verlaufseigenschaften bei folgenden Messwerten:

| | | |
|---|---|---|
| Temperatur (°C)/Vorschub (m/min) | 400/10 | 400/11 |
| Erweichungstemperatur (°C) | 348 | 330 |
| Hitzeschock (°C) | 260 | 260 |
| Bleistifthärte | 4 H | 4 H |
| Aussenfaserdehnung (%) | 67/88 | 67/88 |
| elektr. Durchschlag (kV) | 7,2 | 6,5 |

Die Prüfung erfolgt nach DIN 46 453.

Beispiel 4

In einem 500-ml-Dreihalskolben mit Rührer, Destillationsaufsatz, Innenthermometer und Gasuhr-Anschluss wird bei Raumtemperatur eine Lösung von 75 g 4,4'-Diisocyanatodiphenylmethan, 3 g Phenylisocyanat und 1,0 g Phospholinoxid (Gemisch aus
1-Methyl-1-phospha-2-cyclopenten-1-oxid und
1-Methyl-1-phospha-3-cyclopenten-1-oxid)
in 60 g Toluol und 60 g Chlorbenzol vorgelegt. Innerhalb 1 Std. wird auf 60 °C aufgeheizt, die gemessene Gasmenge beträgt 8,6 l. Es wird 45 Min. bei 55–60 °C nachgerührt. Zu der erhaltenen Polycarbodiimidsuspension (typische Banden im IR bei 2110/2140 $cm^{-1}$) werden 100 g m-Kresol 70 gegeben. Bei 40 °C wird eine Lösung von 44,5 g Maleinsäuremonomethylester (Gesamtsäurezahl 422, Teilsäurezahl 396) und 20 mg Iod in 105 g m-Kresol 70 in die Polycarbodiimidsuspension eingerührt. In ca. 1 Min. steigt die Temperatur auf

76 °C. Nach weiteren 5 Min. beträgt die Temperatur 67 °C und im IR-Spektrum ist die Carbodiimid-Bande bei 2110–2140 $cm^{-1}$ nicht mehr vorhanden. Die klare hellgelbe viskose Reaktionslösung wird innerhalb von 2 Std. unter Abdestillieren des Toluol/Chlorbenzolgemisches auf 180 °C aufgeheizt. Nach weiteren 8 Std. bei 175–180 °C wird kurzzeitig leichtes Vakuum angelegt. Die gesamte Destillatmenge beträgt 118 g. Es werden bei 175 °C 45 g m-Kresol 70 homogen eingerührt, und es wird eine klare rotbraune Polyhydantoinlösung mit einem Festgehalt von ca. 28% (5 Min. bei 360 °C eingebrannt) und einer Viskosität von 1950 mPas bei 20 °C (15%ig in m-Kresol 70) erhalten, die beim Einbrennen auf Prüfblechen bei 360 °C zu hellbraunen elastischen Polymerfilmen führt. Das aus Methanol ausgefällte Polymere zeigt die für in 5-Stellung über Methylenbrücken durch Carbomethoxygruppen substituierten Hydantoine typischen Banden bei 1770, 1720 und 1410 $cm^{-1}$.

Beispiel 5

Eine Lösung von 75 g 4,4'-Diisocyanatodiphenylmethan und 2 g Phenylisocyanat in 60 g Toluol und 80 g Xylol wird bei Raumtemperatur vorgelegt. Es werden 0,5 g Phospholinoxid (Gemisch aus
1-Methyl-1-phospha-2-cyclopenten-1-oxid- und
1-Methyl-1-phospha-3-cyclopenten-1-oxid)
zugesetzt und mit Gasuhr-Anschluss und Rückflusskühler wird innerhalb von 2 Std. bis zur Entwicklung einer Gasmenge von ca. 7 l auf 65–70 °C aufgeheizt. Nach Zugabe von 100 g m-Kresol 70 wird innerhalb von 1 Std. eine Lösung von 98,5 g Maleinsäuremonoisopropylester in 60 g m-Kresol 70 unter intensivem Rühren zugesetzt. Nach Zugabe von weiteren 100 g m-Kresol 70 und 100 mg Toluhydrochinon wird auf 80 °C erhitzt, wobei eine klare viskose Polymerlösung erhalten wird. Unter Abdestillieren von Toluol/Xylol wird die Sumpftemperatur innerhalb von 3 Std. auf 180 °C erhöht. Bei 180 °C wird 6 Std. nachgerührt, wobei gegen Ende Vakuum angelegt und insgesamt eine Destillatmenge von ca. 180 g entfernt wird. Man erhält eine klare braunrote 32,9%ige (bestimmt durch 5-minütiges Einbrennen bei 360 °C) Polymerlösung mit einer Viskosität von 845 mPas (15%ig mit m-Kresol 70 verdünnt bei 20 °C mit Hilfe eines Höppler-Viskosimeters ermittelt), die bei 200, 300 bzw. 360 °C auf Prüfblechen zu klaren elastischen Lackfilmen eingebrannt wird.

Das aus Methanol ausgefällte Polymere wird als braunweisses Pulver erhalten und zeigt die für den Polyhydantoinisopropylester typischen Banden bei 1775, 1720, 1405 und 110 $cm^{-1}$.

Analyse: $(C_{21}H_{20}N_2O_4)_n$ $(364)_n$

| | | | |
|---|---|---|---|
| ber.: | C 69,2 | H 5,5 | N 7,7 |
| gef.: | C 69,4 | H 5,3 | N 7,5 |

Eine Probe des Polyhydantoins wird bei 300 °C unter Stickstoff zu einem harten Formkörper gesintert.

Beispiel 6

Eine Lösung von 150 g 4,4'-Diisocyanatodiphenylmethan und 5 g Phenylisocyanat in 120 g Toluol und 150 g Xylol wird bei Raumtemperatur vorgelegt. Nach Zugabe von 1 g Phospholinoxid (Gemisch aus
1-Methyl-1-phospha-2-cyclopenten-1-oxid- und
1-Methyl-1-phospha-3-cyclopenten-1-oxid)
wird innerhalb von 2 Std. unter Gasuhr-Anschluss auf 70 °C aufgeheizt. Die erhaltene Carbodiimidlösung zeigt die typischen IR-Banden bei 2110/2140 $cm^{-1}$. Nach Zugabe von 200 g m-Kresol 70 wird innerhalb von 1 Std. bei 35–40 °C eine Lösung von 78 g (0,6 Mol) Maleinsäuremonomethylester in 120 g m-Kresol 70 zudosiert. Dann wird innerhalb von 45 Min. auf 60 °C aufgeheizt, wobei eine klare viskose Polymerlösung erhalten wird. Innerhalb von 4 Std. wird unter Abdestillieren von Toluol/Xylol auf 180 °C aufgeheizt. Unter zwischenzeitlicher Zugabe von 90 g Phenol wird 4 Std. bei 180 °C nachgerührt. Danach wird eine viskose Polyhydantoinesterlösung mit den typischen IR-Banden bei 1770, 1710 und 1410 $cm^{-1}$ erhalten, die einen Feststoffgehalt von 30% und eine Viskosität von 860 mPas (15%ig mit m-Kresol 70 verdünnt bei 20 °C in einem Höppler-Viskosimeter gemessen) aufweist. Das Einbrennen ausgestrichener Proben dieser Lacklösung auf Prüfblechen bei 360 °C innerhalb von 5 bzw. 10 Min. führt zu klaren braunroten Lackfilmen mit hoher Elastizität.

Beispiel 7

88,2 g einer 22%igen Diphenylcarbodiimidlösung (0,1 Mol) in Toluol werden bei Raumtemperatur innerhalb 1 Std. in eine vorgelegte Lösung von 14,4 g (0,1 Mol) Fumarsäuremonoethylester in 80 g γ-Butyrolacton eingetropft. Es wird 1 Std. bei 30–35 °C gerührt. Nach Zugabe von 50 mg Toluhydronchinon wird unter Abdestillieren des Toluols auf 180 °C aufgeheizt und 3 Std. bei dieser Temperatur gerührt. Die Reaktionslösung wird im Vakuum eingeengt und das Reaktionsprodukt durch Ausfällen aus einem Wasser/Isopropanol-Gemisch isoliert. Nach dem Trocknen erhält man 23,6 g eines braunweissen Feststoffes, der zur weiteren Reinigung aus Isopropanol/Wasser umkristallisiert wird und einen Fp. von 83–85 °C aufweist. Die Struktur des 1,3-Diphenyl-hydantoyl-5-essigsäureethylesters wird durch die IR/NMR-Spektren und die Elementaranalyse bewiesen.

IR: Hydantoin/Esterbanden 1770/1725/1710/1410 $cm^{-1}$

NMR: $(CDCl_3/DMSO/TMS)$:

| | | | |
|---|---|---|---|
| arom. Protonen | (10 H) | chem. Verschiebung | 7,0-7,5 ppm |
| $-\underline{CH}$ | (1 H,t) | chem. Verschiebung | 5,05 ppm |
| $-O-\underline{CH_2}-$ | (2 H,q) | chem. Verschiebung | 3,16 ppm |
| $-\underline{CH_2}-C-^{o}$ | (2 H,t) | chem. Verschiebung | 2,95 ppm |
| $-\underline{CH_3}$ | (3 H,t) | chem. Verschiebung | 1,1 ppm |

Analyse: $C_{19}H_{18}N_2O_4$ (338)
ber.: C 67,5 H 5,3 N 8,3
gef.: C 67,9 H 5,5 N 8,1

Beispiel 8

225 g einer 19,4%igen Polydiphenylmethancarbodiimidlösung in Methylenchlorid [hergestellt durch Zusatz von 0,5 g Methylphospholinoxid zu 50 g (0,2 Mol) 4,4'-Di-isocyanatodiphenylmethan und 3 g (0,025 Mol) Phenylisocyanat in Methylenchlorid durch Rühren bei Rückflusstemperatur unter Gasuhranschluss bis zur Entwicklung der theoretischen $CO_2$-Menge; im IR-Spektrum wird die Isocyanat-Bande nicht mehr beobachtet, festgestellt wird die typische Carbodiimidbande bei 2140/2110 $cm^{-1}$] wird bei 35 °C vorgelegt. Es werden 100 g γ-Butyrolacton zugegeben, und bei 35–40 °C wird eine Lösung von 26 g (0,2 Mol) Maleinsäuremonomethylester in 10 g Benzylalkohol und 100 g γ-Butyrolacton innerhalb von 30 Min. zugetropft. Nach Zugabe von 100 mg Toluhydrochinon und 20 mg Iod wird unter Abdestillieren des Methylenchlorids in 4 Std. auf 180 °C aufgeheizt, wobei mit 50 mg m-Kresol 70 verdünnt wird. Nach 6 Std. bei 180–185 °C wird eine klare braunrote Polymerlösung mit einem Festgehalt von 21,8% und einer Viskosität von 380 mPas bei 20 °C erhalten. Das aus Methanol aus- und umgefällte Polymere weist die die Hydantoine kennzeichnenden IR-Banden bei 1770–1775/1710–1715 und 1410 $cm^{-1}$ auf. Im NMR ($CDCl^3$/TMS) werden folgende Protonensignale festgestellt:

| | | |
|---|---|---|
| arom. Protonen | (8 H) | chem. Verschiebung 7-7,5 ppm |
| 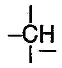 | (1 H) | chem. Verschiebung 4,8 ppm |
| 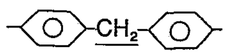 | (2 H) | chem. Verschiebung 3,95 ppm |
| $-O-CH_3$ | (3 H) | chem. Verschiebung 3,55 ppm |
| $-CH_2-$ | (2 H) | chem. Verschiebung 2,97 ppm |

Analyse: $(C_{19}H_{16}N_2O_4)_n$ $(336)_n$
ber. C 67,9 H 4,8 N 8,3
gef. C 68,2 H 4,8 N 8,1

Durch Einbrennen von Proben der erhaltenen Lacklösung auf Prüfblechen werden in 5 Min. bei 360 °C klare elastische Lackfilme erhalten.

Beispiel 9

Eine Poly-Toluylencarbodiimidlösung mit ca. 0,3 Mol Carbodiimidgruppen, hergestellt aus 52,2 g (0,3 Mol) eines Gemisches aus 80% 2,4- und 20% 2,6-Toluylendiisocyanat in 60 g Methylenchlorid und 80 g Xylol in Gegenwart von 3,0 g (0,025 Mol) Phenylisocyanat und 0,5 g Methylphospholinoxid durch Erwärmen auf Rückflusstemperatur bis zum Abklingen der $CO_2$-Entwicklung, wird vorgelegt.

Bei 40–45 °C werden 50 g Phenol und 100 g m-Kresol 70 eingetragen. Anschliessend werden bei 38–42 °C 43,2 g (0,3 Mol) Fumarsäureethylester innerhalb von 30 Min. unter Kühlung portionsweise zugegeben. Es wird eine halbe Stunde bei 40–45 °C nachgerührt und nach Zugabe von 100 mg Toluhydrochinon unter Abdestillieren von Methylenchlorid/Xylol innerhalb von 3 Std. auf 180 °C erhitzt. Bei 180–185 °C wird 4 Std. gerührt, wobei mit steigender Viskosität der Lacklösung mit 50 g Phenol verdünnt wird und insgesamt 152 g Destillat übergehen. Bei 130 °C werden 30 g Xylol homogen eingerührt, und man erhält eine klare braunrote Polymerlösung mit 29,5% Feststoffanteil (5 Min. bei 360 °C) und einer Viskosität von 870 mPas für die mit m-Kresol 70 verdünnte 15%ige Lösung bei 20 °C, die im IR die hydantoinspezifischen Banden bei 1770, 1710 und 1410 $cm^{-1}$ aufweist. Die innerhalb von 5 Min. bei 360 °C auf Prüfblechen eingebrannten klaren elastischen Lackfilme bestätigen die hohen Erweichungstemperaturen der Hydantoinringe enthaltenden Polymeren.

Beispiel 10

Ein Block-Polycarbodiimid der Struktur
–B–B–A–A–A–A–B–B–
aus 50 Mol-% Poly-Diphenylmethancarbodiimid (A) und 50 Mol-% Poly-Naphthylen-1,5-carbodiimid (B), mit ca. 0,4 Mol Carbodiimidgruppen, hergestellt durch stufenweise Carbodiimidisierung von 50 g (0,2 Mol) des Diisocyanats von A und 42 g (0,2 Mol) der Diisocyanats von B unter Zusatz von 3,6 g (0,03 Mol) Phenylisocyanat, 1 g Methylphospholinoxid in 200 g Chlorbenzol durch Erhitzen, wird bei 45–50 °C in dem angegebenen Lösungsmittel vorgelegt.

Nach Zugabe von 165 g Phenol und 100 g m-Kresol 70 werden bei 35–40 °C 52 g (0,4 Mol) Fumarsäuremonomethylester innerhalb von 45 Min. portionsweise eingetragen. Die Reaktion verläuft exotherm. Innerhalb von 4 Std. wird unter Abdestillieren des Chlorbenzols auf 180–185 °C aufgeheizt, die 4 Std. gehalten werden. Die entfernte Destillatmenge beträgt 212 g. Man kühlt auf 130 °C ab, rührt 15 g Xylol homogen ein und erhält eine klare viskose Polymerlösung mit den hydantoinring-spezifischen Banden im IR bei 1770/1710 und 1410/1415 $cm^{-1}$ und einem Feststoffgehalt von 38,2% (5 Min. bei 360 °C). Dieses Einbrennen der Lackproben auf Prüfblechen bei 360 °C führt zu klaren braungelben elastischen Lackfilmen, was die hohen Erweichungstemperaturen dieser Hydantoinringe enthaltenden Polymeren bestätigt. Die Viskosität der mit m-Kresol 70 verdünnten 15%igen Lösung beträgt bei 20 °C 1365 mPas.

Beispiel 11

In eine Lösung aus 200 g (0,8 Mol) 4,4'-Diisocyanatodiphenylmethan in 200 g Methylenchlorid und 200 g Xylol werden bei Raumtemperatur 100 mg Diazabicyclooctan und 15 g Phenol mit ca. 10 ml Toluol eingetragen. Nach ca. 20 minütigem Rühren bei 40–45 °C trägt man 1,5 g Methylphospholinoxid ein und heizt unter Gasuhr-Anschluss auf Rückflusstemperatur auf. Dann wird bei 50–55 °C so lange gerührt, bis eine $CO_2$-Menge von 16,8 l freigesetzt ist. Die Polycarbodiimidlösung weist dann die für Carbodiimidgruppen charakteristischen IR-Absorptionsbanden bei 2135/2105 $cm^{-1}$ auf. Nach dem Abkühlen auf 40 °C werden 300 g Phenol und anschliessend 49,8 g (0,125 Mol) N,N'-Bis-(2-methoxy-carbonyl-2)-4,4'-diamino-diphenylmethan bei 40 bis 45 °C innerhalb von 15 Min. zugegeben. Innerhalb von 30 Min. wird dann unter Kühlung eine Lösung von 24,5 g (0,65 Mol) Maleinsäuremonomethylester in 100 g Phenol eingetragen, wobei mit 10 g Toluol nachgespült wird. Nach weiteren 15 Min. bei 40–45 °C wird die Carbodiimidgruppe im IR nicht mehr festgestellt.

Innerhalb von ca. 1 Std. wird unter Abdestillieren von Lösungsmittel auf 140 °C aufgeheizt. Es wird 1½ Std. bei 145–150 °C gerührt, innerhalb von 2 Std. auf 170 °C erhitzt und 1 Std. bei 170–175 °C gerührt, wobei im Verlauf des Viskositätsanstiegs 200 g Kresol eingetragen werden und insgesamt eine Destillatmenge von 380 g entfernt wird. Man erhält eine klare braunrote Polymerlösung mit den für Hydantoine charakteristischen IR-Banden bei 1765/1705 und 1415 $cm^{-1}$. Der Festgehalt der Lösung beträgt 30,5% (5 Min. bei 360 °C eingebrannt), und die Viskosität wird 15%ig (mit m-Kresol 70 verdünnt bei 20 °C gemessen) zu 2300 mPas bestimmt.

Beispiel 12

1500 g 4,4'-Diisocyanato-diphenylmethan und 36 g Phenylisocyanat werden mit 9 g Methylphospholinoxid als Katalysator in 1500 g Methylenchlorid und 1500 g Toluol bei 40–50 °C zum Polycarbodiimid kondensiert, bis kein Kohlendioxid mehr entweicht. Dann werden bei 45 °C 330 g Phenol und anschliessend unter Kühlung 780 g Maleinsäuremonomethylester eingetragen. Dann wird noch 1 Stunde bei 45–50 °C gerührt, 1740 g einer Mischung aus gleichen Teilen aus Phenol und einem technischen Kresol-Gemisch zugegeben und die Temperatur im Verlaufe von etwa 3 Stunden auf 180 °C gesteigert. Dabei destillieren das Methylenchlorid und das Toluol ab. Anschliessend werden noch 2070 g des Phenol-Kresol-Gemisches eingetragen und danach 4 Stunden bei 180 °C gerührt. Man erhält das Polyhydantoin als ca. 33%ige Lösung mit einer Viskosität von $\eta^{25}$ = 17 400 mPas und den für Hydantoine charakteristischen Banden im IR-Spektrum bei 1717 und 1775 $cm^{-1}$.

Für die Durchführung von Drahtlackerversuchen wird mit einer Mischung aus gleichen Teilen Kresol und Xylol auf einen Festgehalt von 27% herunter verdünnt, was einen Viskositätswert von 230 Sek. (23 °C) ergibt.

Auf einer vertikal angeordneten Lackieranlage mit einem 3 Meter langen Strahlungsofen wird ein Draht von 0,7 mm Durchmesser lackiert.

| Lackierbedingungen: | |
|---|---|
| Ofentemperatur: | 400°C |
| Anzahl der Passagen: | 6 |
| Lackauftragsverfahren: | Düsen |
| Düsenabstufung: | 0,74, 0,76, 0,76, 0,78, 0,78, 0,80 |
| Lackiergeschwindigkeit: | 14 Meter/min |

| Eigenschaften des erhaltenen Lackdrahtes: (DIN 46 453) | |
|---|---|
| Durchmesserzunahme durch die Lackierung: | 58-60 µm |
| Hitzeschock einfacher Durchmesser: | 260°C |
| Erweichungstemperatur: | 380-390°C |
| Bleistifthärte: | 4-5 H |
| Bleistifthärte nach halbstündiger Einwirkung von Ethanol (23°C): | 4-5 H |

Beispiel 13

Aus 250 g 4,4′-Diisocyanato-diphenylmethan und 3 g Phenylisocyanat in 250 g Methylenchlorid und 250 g Toluol wird mit 1,6 g Methylphospholinoxid als Katalysator wie in Beispiel 1 beschrieben eine Lösung des Polycarbodiimids hergestellt. Dann werden bei 50 °C 50 g Phenol und eine Lösung von 158 g Fumarsäuremonoisopropylester in 50 g Toluol/Methylenchlorid (1:1) eingetragen. Anschliessend wird 1 Stunde bei 50 °C nachgerührt, und danach 1 g N,N′-Bis-(dimethylaminoethyl)-methylamin und 320 g Phenol/Kresol (1:1) zugegeben. Die Temperatur des Reaktionsgemisches wird nun im Verlaufe von 4 Stunden auf 180 °C gesteigert, wobei das Methylenchlorid und das Toluol abdestillieren. Dann werden noch 370 g Phenol/Kresol (1:1) zugegeben und 4 Stunden bei 180 °C nachgerührt.

Man erhält eine ca. 33%ige Lösung des Polyhydantoinisopropylesters mit einer Viskosität von 16 560 mPas und den Hydantoinen entsprechenden Banden bei 1710 und 1760 $cm^{-1}$.

Eine Probe der Lacklösung wird wie in Beispiel 1 beschrieben auf einen Kupferdraht appliziert und eingebrannt. Bei einer Lackierungsgeschwindigkeit von 14 m/min beträgt die maximale Aussenfaserdehnung 88%, der Hitzeschock 260 °C und die Erweichungstemperatur ist $>$ 400 °C.

Beispiel 14

In die Lösung eines Polycarbodiimids aus 250 g 4,4′-Diisocyanato-diphenylmethan, 6 g Phenylisocyanat und 1,5 g Methylphospholinoxid in 625 g Methylenchlorid wird nach Zugabe von 0,5 g Triethylendiamin bei 10–20 °C unter Kühlung die Lösung von 130 g Maleinsäuremonomethylester in 125 g Methylenchlorid gerührt, 50 g Phenol zugegeben, die Temperatur auf 50 °C gesteigert und 290 g Phenol/Kresol (1:1) eingetragen. Danach wird unter Abdestillieren des Methylenchlorids das Reaktionsgemisch auf 180 °C aufgeheizt, 340 g Phenol/Kresol (1:1) zugegeben und die Reaktion in 4 Stunden bei 180 °C zu Ende geführt. Man erhält den Polyhydantoinmethylester als klare braune Lösung mit einer Viskosität $\eta^{25}$ von 10 800 mPas. Eine Probe der Lacklösung wird auf eine Glasplatte aufgestrichen und in jeweils 15 min bei 200 und 300 °C zu einem klaren elastischen Lackfilm eingebrannt.

Beispiel 15

In eine Lösung von 147 g Maleinsäuremonomethylester in 300 g Methylenchlorid und 50 g Phenol wird bei 50 °C eine Polycarbodiimid-Lösung aus 250 g 4,4′-Diisocyanatodiphenylmethan und 1,5 g Methylphospholinoxid in 500 g Methylenchlorid eingetragen. Dann wird 1 Stunde bei 50 °C gerührt, 290 g Phenol/Kresol (1:1) zugegeben, die Temperatur unter Abdestillieren des Methylenchlorids auf 180 °C gesteigert und nochmals 240 g Phenol/Kresol zugegeben. Nach 4 Stunden bei 180 °C erhält man eine Lösung des Poly-hydantoinmethylesters mit einer Viskosität $\eta^{25}$ und 5300 mPas und Hydantoin-Banden bei 1710 und 1770 $cm^{-1}$.

Eine Probe des Hydantoinlösung wird auf ein Prüfblech aufgestrichen und zuerst bei 200 °C und dann bei 300 °C zu einem klaren elastischen Lackfilm eingebrannt.

Beispiel 16

In eine Polycarbodiimid-Lösung aus 250 g 4,4′-Diisocyanato-diphenylmethan, 6 g Phenylisocyanat und 1,5 g Methylphospholinoxid in 250 g Methylenchlorid, 250 g Toluol und 57 g Caprolactam werden unter Kühlung bei 45–50 °C 130 g Maleinsäuremonomethylester eingetropft. Dann wird 30 min bei dieser Temperatur gerührt, 50 g Phenol zugegeben und nach nochmals 30 min 290 g Phenol/Kresol (1:1) zugegeben. Anschliessend wird die Temperatur unter Abdestillieren des Methylenchlorids und Toluols im Verlaufe von 4 Stunden auf 180 °C gesteigert, 340 g Phenol/Kresol eingetragen und 4 Stunden bei 180 °C nachgerührt. Man erhält den Phenol-hydantoinmethylester als ca. 33%ige braune Lösung mit der Viskosität $\eta^{25}$ = 11 600 mPas.

Eine Probe der Lacklösung wird auf eine Glasplatte aufgestrichen und bei 200 °C und 300 °C zu einem klaren elastischen Lackfilm eingebrannt.

Beispiel 17

In die Lösung eines Carbodiimids aus 250 g 4,4′-Diisocyanato-diphenylmethan, 6 g Phenylisocyanat und 1,5 g Methylphospholinoxid in 250 g Methylenchlorid und 250 g Toluol werden 12 g Methanol eingetropft und die Lösung dann 2 Stunden bei 50 °C gehalten. Nach der Zugabe von 50 g Phenol werden bei 45 °C unter Kühlung 130 g Maleinsäuremonomethylester eingetragen, 1 Stunde bei 50 °C gerührt und danach 290 g Phenol/Kresol zugegeben. Anschliessend wird das Reaktionsgemisch unter Abdestillieren des Methylenchlorids und Toluols auf 180 °C aufgeheizt, nochmals 340 g Phenol/Kresol (1:1) zugegeben und die Kondensation in 4 Stunden bei 180 °C zu Ende geführt. Man erhält eine 33%ige Polyhydantoinmethylester-Lösung mit einer Viskosität $\eta^{25}$ = 7020 mPas. Eine Probe der Lacklösung wird bei 200 °C und 300 °C auf einer Glasplatte zu einem klaren elastischen Lackfilm eingebrannt.

Beispiel 18

In eine Polycarbodiimid-Lösung aus 250 g 4,4′-Diisocyanato-diphenylmethan, 3 g Phenylisocyanat und 1,5 g Methylphospholinoxid in 250 g Methylenchlorid, 250 g Toluol und 50 g Phenol wird bei 45 °C eine Lösung von 206 g Maleinsäuremonobenzylester in 100 g Methylenchlorid eingetropft. Dann wird noch 30 min bei dieser Temperatur gerührt, 310 g Phenol/Kresol (1:1) zugegeben und in 1,5 Stunden auf 180 °C aufgeheizt. Man gibt nun 360 g Phenol/Kresol zu, rührt noch 4 Stunden bei 180 °C nach und erhält eine 33%ige Lösung des Polyhydantoinbenzylesters mit einer Viskosität $\eta^{25}$ von 6200 mPas.

Eine Probe der Lacklösung wird auf einem Prüfblech bei 200 °C und 300 °C zu einem klaren elastischen Lackfilm eingebrannt.

**Patentansprüche**

1. Verfahren zur Herstellung von mindestens einen Hydantoinring enthaltenden Verbindungen, dadurch gekennzeichnet, dass man ein Carbodiimid mit einem Derivat einer α,β-ungesättigten Carbonsäure der Formel

$$HOOC\text{–}CH = CH - R^5$$

worin $R^5$ Benzoyl ($COC_6H_5$), $COOR^6$ oder eine Säureamidgruppe $-CONR^7R^8$ bedeutet,
worin
$R^6$ Benzyl, Cycloalkyl, Alkenyl und Alkinyl bedeutet und
$R^7$ und $R^8$ Alkyl bedeuten
bei Temperaturen von −20 bis 250 °C, gegebenenfalls in einem Lösungsmittel und gegebenenfalls in Anwesenheit eines Katalysators umsetzt.

2. Verfahren nach Anspruch 1, dadurch gekennzeichnet, dass Methylenchlorid oder ein Methylenchlorid enthaltendes Lösungsmittelgemisch als Lösungsmittel verwendet wird.

**Claims**

1. Process for the production of compounds containing at least one hydantoin ring, characterised in that a carbodiimide is reacted with a derivative of an α,β-unsaturated carboxylic acid of the formula

$$HOOC\text{–}CH = CH\text{–}R^5$$

wherein
$R^5$ denotes benzoyl ($COC_6H_5$), $COOR^6$ or an acid amide group $-CONR^7R^8$,
wherein
$R^6$ denotes benzyl, cycloalkyl, alkenyl and alkinyl and
$R^7$ and $R^8$ denote alkyl,
at temperatures of −20 to 250 °C, if appropriate in a solvent and if appropriate in the presence of a catalyst.

2. Process according to Claim 1, characterised in that methylene chloride or a mixture of solvents containing methylene chloride is used as the solvent.

**Revendications**

1. Procédé de préparation de composés contenant au moins un cycle hydantoïne, caractérisé en ce que l'on fait réagir un carbodiimide avec un dérivé d'un acide carboxylique α,β-insaturé de formule

$$HOOC\text{–}CH = CH\text{–}R^5$$

dans laquelle
$R^5$ représente un groupe benzoyle ($COC_6H_5$), $COOR^6$ ou un groupe amide $-CONR^7R^8$
dans lesquels
$R^6$ représente un groupe benzyle, cycloheptyle, alcényle ou alcynyle et
$R^7$ et $R^8$ représentent des groupes alkyle,
à des températures de −20 à 250 °C, éventuellement dans un solvant et eventuellement en présence d'un catalyseur.

2. Procédé selon la revendication 1, caractérisé en ce que l'on utilise en tant que solvant le chlorure de méthylène ou un mélange de solvants contenant du chlorure de méthylène.